# EUROPEAN PATENT APPLICATION

(11) **EP 4 563 127 A1**
(43) Date of publication of application: **04.06.2025**
(21) Application number: 23849791.1
(22) Date of filing: 27.06.2023
(51) Int. Cl.: A61F 5/37, A61B 17/42

(54) **RING FOR PREVENTING ORGAN PROLAPSE**

(30) Priority: 03.08.2022 JP 2022123867
(71) Applicant: Kitazato Corporation, Fuji-shi, Shizuoka 416-0932 (JP)
(72) Inventor: INOUE, Futoshi, Fuji-shi, Shizuoka 416-0932 (JP)
(74) Representative: Casalonga
(86) International application number: PCT/JP2023/023688
(87) International publication number: WO 2024/029232

(57) **Abstract**

An organ prolapse preventing ring (1) for intravaginal insertion includes a ring-shaped portion (2) constituted by a frame portion having a circular cross section. The ring-shaped portion includes a ring body (21) formed of a soft material and four arcuate reinforcing members (3a), (3b), (3c), and (3d) formed of a hard or semi-hard material and embedded in the ring body (21). The ring-shaped portion (2) includes a first bendable portion (51) composed of a first pair of arcuate-reinforcing-member absent portions (5a) and (5c) formed of only the soft material and facing each other, and a second bendable portion (52) composed of a second pair of arcuate-reinforcing-member absent portions that are formed of only the soft material, orthogonal to the first pair of arcuate-reinforcing-member absent portions (5a) and (5c), and face each other.

## Description

### Technical field

The present invention relates to an organ prolapse preventing ring for intravaginal insertion configured to prevent prolapse of an organ such as uterine prolapse and bladder prolapse.

### Background Art

Elderly women often develop uterine prolapse and bladder prolapse. The force of tissues hanging the uterus upward and the force of muscles supporting the uterus from below decline with age, the uterus descends from a predetermined position, and a portion of the uterus and the bladder wall near the uterus may be exposed to the outside of the body, which is the uterine prolapse and the bladder prolapse. Vaginal hysterectomy is desirable for the treatment of the uterine prolapse and the bladder prolapse, but vaginal hysterectomy is rarely performed because it is technically difficult.

Therefore, there is demand for a conservative treatment of the uterine prolapse and the bladder prolapse that can be performed without surgery.

JP 2002-085436A (Patent Document 1) proposes an organ prolapse preventing ring for intravaginal insertion configured to prevent prolapse of an organ such as the uterine prolapse and the bladder prolapse. The ring 1 for preventing the uterine prolapse and the bladder prolapse includes an annular portion 2 and a flexible curved rod-shaped portion 3 provided such that a starting end and a terminal end thereof are connected to the annular portion 2 and an intermediate portion 3a between the starting end and the terminal end extends above an annular plane formed by the annular portion 2 toward the center. The annular portion 2 has an opening 4 formed by partially cutting out the annular portion 2.

Also, a pessary for the treatment of uterine prolapse is proposed in JP 2011-167364A (Patent Document 2). The pessary 10 for the treatment of uterine prolapse proposed in Patent Document 2 is characterized by being constituted by a cylindrical sheet-shaped member 11 that is not deformable in an axial direction but is deformable in a radial direction. The sheet-shaped member 11 includes a plurality of metal pieces 13 discontinuously provided in a circumferential direction with gaps 12 having a predetermined length therebetween and a silicone portion 14 continuously provided in the circumferential direction to coat each metal piece 13. Silicone portions 14a provided in the gaps 12 are deformable in the radial direction.

### Prior Art Documents

### Patent Documents

[Patent Document 1] JP 2002-085436A
[Patent Document 2] JP 2011-167364A (WO 2011-102158A, US 2012-289771A, EP 2537493A)

### Summary of Invention

### Technical Problem

When the ring proposed in Patent Document 1 is obliquely inserted into the vagina such that one end of the ring is located in an inner part of the uterus and a lower part of the bladder is located on the curved rod-shaped portion, the uterus can be pushed up from below by the annular portion. The bladder can be supported from below by the curved rod-shaped portion, which is effective in mitigating the symptoms of the uterine prolapse and the bladder prolapse without causing surgical stress.

However, the ring proposed in Patent Document 1 cannot be easily deformed to become smaller when it is to be inserted into the vagina.

The pessary proposed in Patent Document 2 can be deformed, but when the pessary is deformed, two end portions of the pessary need to be held down simultaneously, and the pessary needs to be inserted into the vagina in this state, and the insertion is not easy. Also, the pessary is constituted by a cylindrical sheet with a small thickness, and therefore, it is not easy to take out the pessary from the vagina after it is inserted into the vagina.

An object of the present invention is to provide an organ prolapse preventing ring for intravaginal insertion, which can be easily inserted into the vagina and favorably taken out from the vagina.

### Solution to Problem

The above object is achieved by the following.

An organ prolapse preventing ring for intravaginal insertion, the ring including a ring-shaped portion formed by a frame portion having a circular or oval cross section, wherein the ring-shaped portion includes a ring body formed of a soft material and four arcuate reinforcing members formed of a hard or semi-hard material and embedded in the ring body, the four arcuate reinforcing members are spaced apart from each other, and the ring-shaped portion includes a first pair of arcuate-reinforcing-member absent portions that are formed of only the soft material and face each other, a first bendable portion composed of the first pair of arcuate-reinforcing-member absent portions, a second pair of arcuate-reinforcing-member absent portions that are formed of only the soft material, orthogonal to the first pair of arcuate-reinforcing-member absent portions, and face each other, and a second bendable portion composed of the second pair of arcuate-reinforcing-member absent portions.

### Brief Description of Drawings

FIG. 1 is a front view of an organ prolapse preventing ring according to an embodiment of the present invention.
FIG. 2 is a right side view of the organ prolapse preventing ring shown in FIG. 1.
FIG. 3 is a perspective view of the organ prolapse preventing ring shown in FIG. 1.
FIG. 4 is a cross-sectional view taken along a line A-A in FIG. 1.
FIG. 5 is a cross-sectional view taken along a line B-B in FIG. 1.
FIG. 6 is a cross-sectional view taken along a line C-C in FIG. 1.
FIG. 7 is a cross-sectional view taken along a line D-D in FIG. 2.
FIG. 8 is a front view of an arcuate reinforcing member used for an organ prolapse preventing ring according to an embodiment of the present invention.
FIG. 9 is a bottom view of the arcuate reinforcing member shown in FIG. 8.
FIG. 10 is a front view of an organ prolapse preventing ring according to another embodiment of the present invention.
FIG. 11 is a bottom view of the organ prolapse preventing ring shown in FIG. 10.
FIG. 12 is a left side view of the organ prolapse preventing ring shown in FIG. 10.
FIG. 13 is a perspective view of the organ prolapse preventing ring shown in FIG. 10.
FIG. 14 is a cross-sectional view taken along a line G-G in FIG. 12.
FIG. 15 is a cross-sectional view taken along a line E-E in FIG. 10.
FIG. 16 is a cross-sectional view taken along a line F-F in FIG. 10.
FIG. 17 is a front view of another example of the arcuate reinforcing member used for an organ prolapse preventing ring according to an embodiment of the present invention.
FIG. 18 is a bottom view of the arcuate reinforcing member shown in FIG. 17.
FIG. 19 is a front view of an organ prolapse preventing ring according to another embodiment of the present invention.
FIG. 20 is a right side view of the organ prolapse preventing ring shown in FIG. 19.
FIG. 21 is a perspective view of the organ prolapse preventing ring shown in FIG. 19.
FIG. 22 is a cross-sectional view taken along a line H-H in FIG. 19.
FIG. 23 is a cross-sectional view taken along a line J-J in FIG. 19.
FIG. 24 is a cross-sectional view taken along a line K-K in FIG. 19.
FIG. 25 is a cross-sectional view taken along a line L-L in FIG. 20.
FIG. 26 is a front view of an organ prolapse preventing ring according to another embodiment of the present invention.
FIG. 27 is a bottom view of the organ prolapse preventing ring shown in FIG. 26.
FIG. 28 is a right side view of the organ prolapse preventing ring shown in FIG. 27.
FIG. 29 is a cross-sectional view taken along a line M-M in FIG. 26.
FIG. 30 is a cross-sectional view taken along a line N-N in FIG. 28.

### Description of Embodiments

The following describes an organ prolapse preventing ring for intravaginal insertion according to the present invention with reference to the embodiments shown in the drawings.

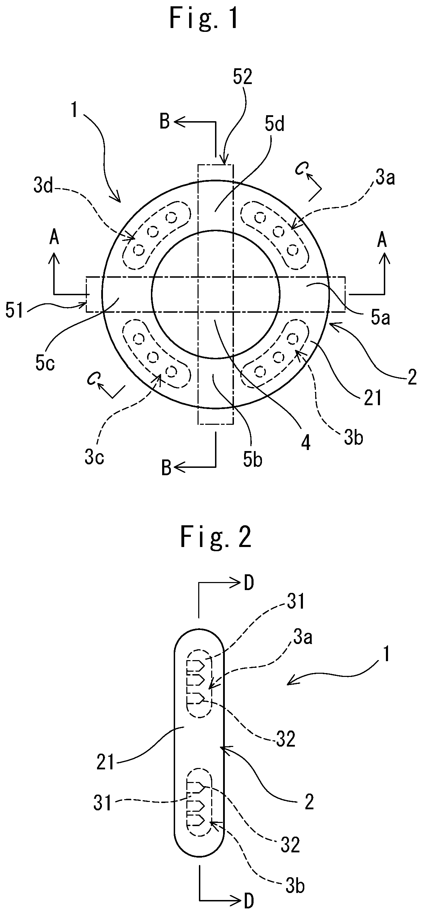

An organ prolapse preventing ring 1 for intravaginal insertion according to the present invention includes a ring-shaped portion 2 formed by a frame portion having a circular or oval cross section as shown in FIGS. 1 to 9. The ring-shaped portion 2 includes a ring body 21 formed of a soft material and four arcuate reinforcing members 3a, 3b, 3c, and 3d that are formed of a hard or semi-hard material and embedded in the ring body 21, and the arcuate reinforcing members 3a, 3b, 3c, and 3d are spaced apart from each other.

The ring-shaped portion 2 includes a first pair of arcuate-reinforcing-member absent portions 5a and 5c that are formed of only the soft material and face each other, a first bendable portion 51 composed of the first pair of arcuate-reinforcing-member absent portions 5a and 5c, a second pair of arcuate-reinforcing-member absent portions 5b and 5d that are formed of only the soft material, orthogonal to the first pair of arcuate-reinforcing-member absent portions 5a and 5c, and face each other, and a second bendable portion 52 composed of the second pair of arcuate-reinforcing-member absent portions 5b and 5d.

This organ prolapse preventing ring for intravaginal insertion includes the first bendable portion and the second bendable portion that can be bent in a direction different from a bendable direction of the first bendable portion. Accordingly, the organ prolapse preventing ring can be bent in both of the two bendable portions, and therefore, the bending operation can be easily performed. Moreover, two side portions of the ring project in the bent state, and the ring can be inserted into the vagina from one of the two side portions, and therefore, the ring can be easily inserted into the vagina. Furthermore, the ring-shaped portion is formed by the frame portion having a circular or oval cross section, and therefore, it is possible to favorably take out the ring from the vagina by hooking a finger on the ring-shaped portion.

As shown in FIGS. 1 to 7, the organ prolapse preventing ring 1 for intravaginal insertion according to this embodiment includes the ring-shaped portion 2 formed by the frame portion having a circular or oval cross section. The ring-shaped portion 2 includes the ring body 21 formed of a soft material and the four arcuate reinforcing members 3a, 3b, 3c, and 3d formed of a hard material and embedded in the ring body 21.

The ring-shaped portion 2 is an annular member having a substantially perfect circular shape, and has a central opening 4. The shape of the ring-shaped portion 2 is not limited to a perfect circular shape, and may also be an oval shape, a deformed circular shape, or the like. Although the size of the ring-shaped portion 2 varies depending on the patient's physique or the like, the diameter (outer diameter) of the ring-shaped portion 2 is preferably about 40 to 120 mm, and particularly preferably 60 to 100 mm. The inner diameter of the ring-shaped portion 2 (the diameter of the central opening 4) is preferably about 30 to 110 mm, and particularly preferably 35 to 95 mm.

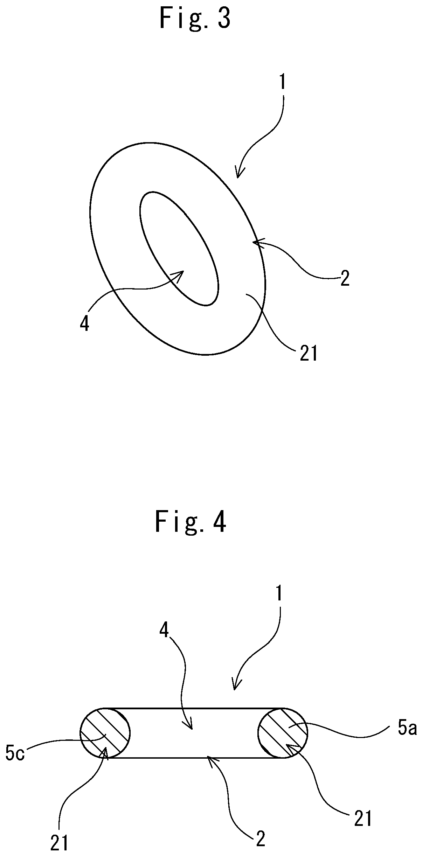

The ring-shaped portion 2 is formed by the frame portion having a circular or oval cross section as shown in FIGS. 4, 5, and 6. The diameter of the frame portion forming the ring-shaped portion 2 is preferably 8 to 16 mm, and particularly preferably 10 to 14 mm. The entire frame portion forming the ring-shaped portion 2 need not necessarily have the same thickness (diameter). For example, the diameter of the arcuate-reinforcing-member absent portions, which will be described later, may be smaller or larger than the diameter of other portions (arcuate-reinforcing-member present portions).

The ring-shaped portion 2 is formed by the ring body 21 and the four arcuate reinforcing members 3a, 3b, 3c, and 3d embedded in the ring body 21. The four arcuate reinforcing members 3a, 3b, 3c, and 3d are arranged at equal angular (90 degrees) intervals with respect to the center of the ring-shaped portion 2. Adjacent ones of the arcuate reinforcing members 3a, 3b, 3c, and 3d are spaced apart from each other by a predetermined arc length. The four arcuate reinforcing members 3a, 3b, 3c, and 3d are preferably the same as each other.

The four arcuate-reinforcing-member absent portions 5a, 5b, 5c, and 5d are formed in regions spacing the four arcuate reinforcing members 3a, 3b, 3c, and 3d apart from each other. The four arcuate-reinforcing-member absent portions 5a, 5b, 5c, and 5d are formed of only the soft material forming the ring body 21. The ring body 21 forms the entire outer shape of the ring-shaped portion 2, and the arcuate reinforcing members 3a, 3b, 3c, and 3d are not exposed to the outer surface of the ring-shaped portion 2.

The ring-shaped portion 2 includes: the first bendable portion 51 composed of the first pair of arcuate-reinforcing-member absent portions 5a and 5c formed of only the soft material and facing each other; and the second bendable portion 52 composed of the second pair of arcuate-reinforcing-member absent portions 5b and 5d. The second pair of arcuate-reinforcing-member absent portions 5b and 5d are orthogonal to the first pair of arcuate-reinforcing-member absent portions 5a and 5c. Accordingly, the second bendable portion 52 can be bent in a direction different from the bendable direction of the first bendable portion 51. Specifically, the second bendable portion 52 can be bent in a direction substantially orthogonal to the bendable direction of the first bendable portion 51. It is particularly preferable that the second bendable portion 52 can be bent in a direction orthogonal to the bendable direction of the first bendable portion 51.

The central angle of an arc formed by each of the arcuate-reinforcing-member absent portions 5a, 5b, 5c, and 5d is preferably 24 to 45 degrees, and particularly preferably 25 to 40 degrees. The length of the outer edge of the arc formed by each of the arcuate-reinforcing-member absent portions 5a, 5b, 5c, and 5d is preferably 1/15 to 5/40 of the outer circumference (circumferential length) of the ring-shaped portion 2, and particularly preferably 1/14 to 1/9 of the outer circumference of the ring-shaped portion 2.
The four arcuate reinforcing members 3a, 3b, 3c, and 3d formed of the hard material are embedded in the ring body 21 and spaced apart from each other. The four arcuate reinforcing members 3a, 3b, 3c, and 3d are arranged at equal angular (90 degrees) intervals with respect to the center of the ring-shaped portion 2. The central angle of an arc formed by each of the arcuate reinforcing members 3a, 3b, 3c, and 3d is preferably 40 to 72 degrees, and particularly preferably 45 to 65 degrees. The length of the arc formed by each of the arcuate reinforcing members 3a, 3b, 3c, and 3d is preferably 11/100 to 2/10 of the circumferential length of the corresponding portion (circular portion where the four arcuate reinforcing members are located), and particularly preferably 5/40 to 18/100 of the circumferential length of the corresponding portion.

The arcuate reinforcing members 3a, 3b, 3c, and 3d each include a body 31 and a recess 32 extending from one surface side toward another surface side of the ring-shaped portion 2. In this embodiment, the recess 32 includes a main portion 32a extending a predetermined length with a substantially cylindrical shape (the same diameter) and a distal end portion 32b (a conical distal end) with a reduced diameter. The entire recess 32 is filled with the soft material that forms the ring body 21.

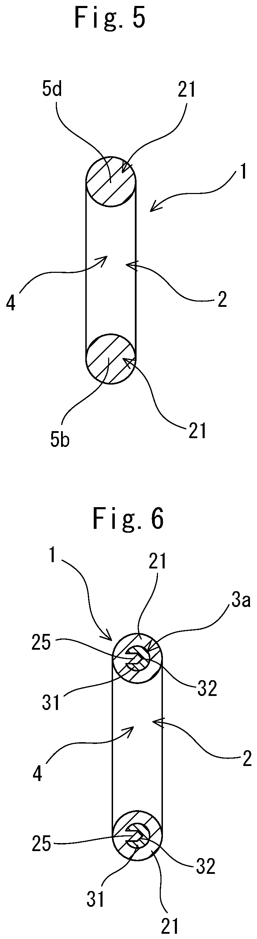

The arcuate reinforcing members 3a, 3b, 3c, and 3d have a circular cross section as shown in FIG. 6, except for the recess 32. The cross section may also be oval. The diameter of the arcuate reinforcing members 3a, 3b, 3c, and 3d (excluding the recess) is preferably 1/3 to 2/3 of the diameter of the frame portion forming the ring-shaped portion 2, and particularly preferably 2/5 to 3/5 of the diameter of the frame portion.

The radius of curvature of the arcuate reinforcing members 3a, 3b, 3c, and 3d is preferably equivalent to the radius of curvature of the corresponding portion (circular portion where the four arcuate reinforcing members are located) of the ring-shaped portion 2, and particularly preferably the same as the radius of curvature of the corresponding portion.

It is preferable that the four arcuate reinforcing members are arranged at approximately equal angular intervals with respect to the center of the ring-shaped portion, the central angle of the arc formed by each of the four arcuate reinforcing members is 40 to 72 degrees, and the central angle of the arc formed by each of the four arcuate-reinforcing-member absent portions is 24 to 45 degrees.

It is particularly preferable that the four arcuate reinforcing members are arranged at approximately equal angular intervals with respect to the center of the ring-shaped portion, the central angle of the arc formed by each of the four arcuate reinforcing members is 45 to 65 degrees, and the central angle of the arc formed by each of the four arcuate-reinforcing-member absent portions is 25 to 40 degrees.

As described above, the organ prolapse preventing ring 1 according to this embodiment includes the first bendable portion 51 formed of only the soft material and the second bendable portion 52 formed of only the soft material and composed of the second pair of arcuate-reinforcing-member absent portions 5b and 5d that are orthogonal to the first pair of arcuate-reinforcing-member absent portions 5a and 5c (the first bendable portion 51) and face each other.

Accordingly, the organ prolapse preventing ring 1 can be folded in half such that two end portions (the first pair of arcuate-reinforcing-member absent portions 5a and 5c) slightly bulge by bending the first bendable portion 51 formed of only the soft material along its center line, which serves as a bending axis, that is, by bringing the second pair of arcuate-reinforcing-member absent portions 5b and 5d close to each other or into contact with each other. Similarly, the organ prolapse preventing ring 1 can be folded in half such that two end portions (the second pair of arcuate-reinforcing-member absent portions 5b and 5d) slightly bulge by bending the second bendable portion 52 formed of only the soft material along its center line, which serves as a bending axis, that is, by bringing the first pair of arcuate-reinforcing-member absent portions 5a and 5c close to each other or into contact with each other.

As described above, the organ prolapse preventing ring 1 can be bent in two directions, and accordingly, an operation for finding the bendable portions is considerably easy and substantially unnecessary, and the bending operation is easy when compared with a ring that includes only one bendable portion. Moreover, the first bendable portion 51 and the second bendable portion 52 are formed of only the soft material forming the ring body 21, and accordingly, these portions can be easily bent.

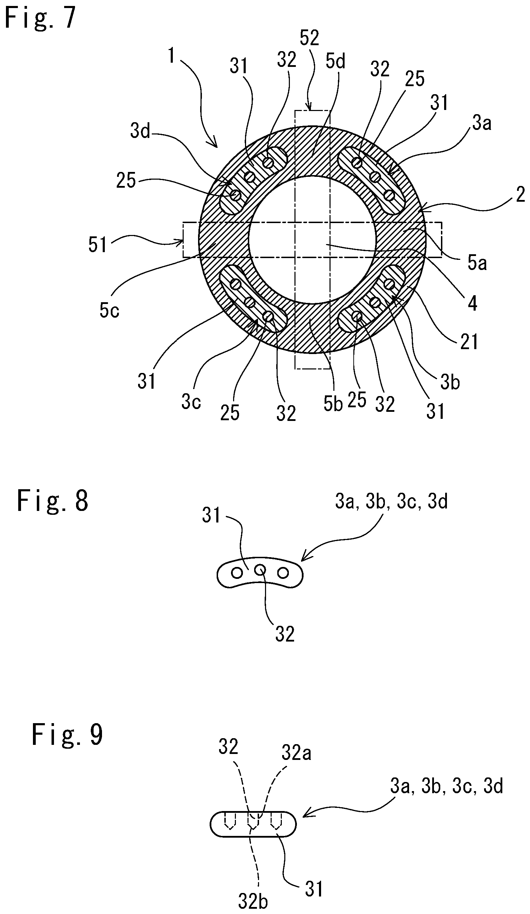

In the organ prolapse preventing ring 1 according to this embodiment, the arcuate reinforcing members 3a, 3b, 3c, and 3d each include the main body 31 and the recess 32 extending from one surface side toward another surface side of the ring-shaped portion 2 (main body 31) as shown in FIGS. 6 to 9. The recess 32 is filled with a soft material 25 forming the ring body 21 as shown in FIGS. 6 and 7. This prevents the arcuate reinforcing members 3a, 3b, 3c, and 3d from moving in the ring-shaped portion 2 while the organ prolapse preventing ring 1 is being bent and after the organ prolapse preventing ring 1 is inserted into the vagina. The diameter of the recess 32 is preferably 2/10 to 6/10 of the diameter of the arcuate reinforcing members 3a, 3b, 3c, and 3d, and particularly preferably 3/10 to 5/10 of the diameter of the arcuate reinforcing members 3a, 3b, 3c, and 3d. The depth of the recess 32, particularly in this embodiment, the depth of the portion 32a extending with the same diameter is preferably 40/100 to 75/100 of the diameter of the arcuate reinforcing members 3a, 3b, 3c, and 3d, and particularly preferably 45/100 to 65/100 of the diameter of the arcuate reinforcing members 3a, 3b, 3c, and 3d.

In the organ prolapse preventing ring 1 according to this embodiment, the arcuate reinforcing members 3a, 3b, 3c, and 3d each preferably include a plurality of recesses 32. It is particularly preferable that the arcuate reinforcing members 3a, 3b, 3c, and 3d each include recesses 32 in two end portions. In this embodiment, the arcuate reinforcing members 3a, 3b, 3c, and 3d each include recesses 32 at three positions, i.e., in the two end portions and a central portion. All the recesses are filled with the soft material 25 forming the ring body 21.

A soft elastic material is suitable as the soft material (ring body forming material) used for the ring body 21. Specifically, soft rubber or a soft elastomer is used. Preferable examples of the soft rubber include natural rubber, isoprene rubber, butyl rubber, chloroprene rubber, nitrile-butadiene rubber, styrene-butadiene rubber, and silicone rubber, and vulcanized soft rubber is particularly preferable. Preferable examples of the soft elastomer include a polyvinyl chloride-based elastomer, a polyolefin-based elastomer, a styrene-based elastomer, a polyester-based elastomer, a polyamide-based elastomer, a polyurethane-based elastomer, and a silicone-based elastomer.

Also, a soft thermoplastic elastomer is preferable as the soft elastomer. It is possible to use, as the thermoplastic elastomer, a polyester-based elastomer (e.g., a polyethylene terephthalate elastomer), a nylon-based elastomer (e.g., a polyamide elastomer), a urethane-based elastomer (e.g., a polyurethane elastomer), an olefin-based elastomer (e.g., a polyethylene elastomer or a polypropylene elastomer), a fluororesin-based elastomer, or the like.

Among the soft elastic materials listed above, silicone rubber (a crosslinked silicone elastomer) has suitable elastic properties and strength and thus is suitable. Preferable silicone rubber is millable silicone rubber. The millable silicone rubber is a silicone rubber compound that is similar to unvulcanized compounded rubber of natural rubber or ordinary synthetic rubber before curing, and can be plasticized and mixed in a milling roll machine, a sealed mixer, or the like. In particular, crosslinked millable silicone rubber is preferable as the soft elastic material.

As the millable silicone rubber, it is possible to use, for example, KE-541-U, KE-561-U, KE-931-U, KE-941-U, KE-951-U, or KE-961-U manufactured by Shin-Etsu Chemical Co., Ltd. One of these types of silicone rubber may be used alone, or two or more of them may be used in combination. A crosslinking agent is added to the millable silicone rubber. As the crosslinking agent, an organic peroxide vulcanizing agent, specifically, C-23N (containing p-methylbenzoyl peroxide) manufactured by Shin-Etsu Chemical Co., Ltd., or a platinum-based catalyst, specifically, C-25A manufactured by Shin-Etsu Chemical Co., Ltd., is suitably used, for example.

Also, a durometer hardness A at 25 degrees C, which is defined in accordance with JIS K6253 "Method for testing hardness of vulcanized rubber and thermoplastic rubber", of the ring body forming material (soft elastic material) is preferably 15 to 50, more preferably 20 to 45, and particularly preferably 25 to 40. In this case, it is possible to favorably bend the first bendable portion 51 composed of the first pair of arcuate-reinforcing-member absent portions 5a and 5c that are formed of only the soft material and face each other, and it is possible to favorably bend, in a direction different from (specifically, a direction substantially orthogonal to) the bendable direction of the first bendable portion 51, the second bendable portion 52 composed of the second pair of arcuate-reinforcing-member absent portions 5b and 5d that are formed of only the soft material, orthogonal to the first pair of arcuate-reinforcing-member absent portions 5a and 5c, and face each other. It is preferable that the second bendable portion 52 can be bent in a direction orthogonal to the bendable direction of the first bendable portion 51.

Also, elongation at break of the soft elastic material measured in accordance with JIS K6251 is preferably 300 to 900%, and particularly preferably 400 to 800%. The tensile strength measured in accordance with JIS K6251 is preferably 5 to 12 MPa, and particularly preferably 6 to 10 MPa.

It is preferable that the ring body forming material (soft elastic material) is transparent or translucent and the embedded arcuate reinforcing members 3a, 3b, 3c, and 3d are visible. In this case, the arcuate-reinforcing-member absent portions 5a, 5b, 5c, and 5d can be confirmed and the positions of the first bendable portion 51 and the second bendable portion 52 can be confirmed easily.

A hard or semi-hard material is used to form the arcuate reinforcing members 3a, 3b, 3c, and 3d. Specifically, a hard or semi-hard thermoplastic resin is suitable. Specifically, it is preferable to use a synthetic resin such as polyamide (nylon 6 or nylon 66), polyvinylidene fluoride, polycarbonate, acrylic resin (e.g., polyacrylate, specifically, PMMA (poly methyl methacrylate), polyacrylamide, an acrylonitrile-styrene copolymer, an acrylonitrile-butadiene-styrene copolymer, etc.), polyester (e.g., polyethylene terephthalate or polybutylene terephthalate), polyolefin (e.g., polyethylene, polypropylene, or an ethylene-propylene copolymer), styrene-based resin [e.g. polystyrene or MS resin (a methacrylate-styrene copolymer)], MBS resin (a methacrylate-butylene-styrene copolymer), polyvinyl chloride (hard vinyl chloride), polysulfone, or polyarylate, and polyamide (nylon 6 or nylon 66) is particularly preferable. Also, it is preferable that the material forming the arcuate reinforcing members 3a, 3b, 3c, and 3d is colored (other than white).

The tensile modulus (ISO 527-1, 23 degrees C) of the material forming the arcuate reinforcing members is preferably 1000 to 4000 MPa, and particularly preferably 1500 to 3000 MPa. The flexural modulus (ISO 178, 23 degrees C) is preferably 1000 to 4000 MPa, and particularly preferably 1500 to 3000 MPa. The flexural strength (ISO 178, 23 degrees C) is preferably 15 to 45 MPa, and particularly preferably 20 to 40 MPa.

When the material forming the arcuate reinforcing members is a thermoplastic resin and the material used to form the ring body is crosslinked silicone rubber, it is preferable that the melting point of the thermoplastic resin is higher than a heating temperature (crosslinking treatment temperature) for crosslinking. In particular, when the material used to form the ring body is crosslinked silicone rubber, the melting point of the material forming the arcuate reinforcing members is preferably 15 degrees C or more higher than the heating temperature for crosslinking. The melting point of the thermoplastic resin forming the arcuate reinforcing members is preferably 240 to 280 degrees C, and particularly preferably 245 to 275 degrees C, although the preferable melting point varies depending on the crosslinking treatment temperature of the soft elastic material that is used.

It is also preferable that the material forming the arcuate reinforcing members and the ring body forming material are not miscible with each other. If these materials are not miscible with each other, the arcuate reinforcing members and the ring body do not fuse at contact surfaces and do not adhere to each other at interfaces. Accordingly, when the organ prolapse preventing ring 1 is deformed, the arcuate reinforcing members can slightly slide in the ring body, facilitating deformation of the organ prolapse preventing ring. This state can be realized by selecting polyamide (nylon 6 or nylon 66) as the material forming the arcuate reinforcing members and selecting silicone rubber (crosslinked silicone elastomer) as the ring body forming material, for example.

The thickness of the soft material of the ring body 21 covering outer surfaces of the arcuate reinforcing members 3a, 3b, 3c, and 3d is preferably 1.5 mm or more, particularly preferably 2 mm or more, and even more preferably 2.5 mm or more. In this case, the organ prolapse preventing ring 1 has sufficient surface elasticity in the portions where the arcuate reinforcing members 3a, 3b, 3c, and 3d are arranged.

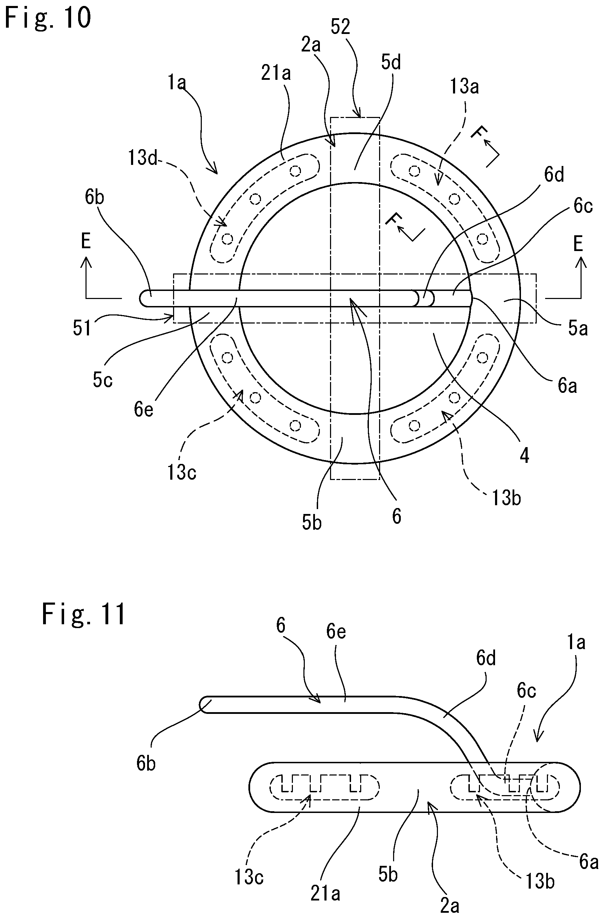

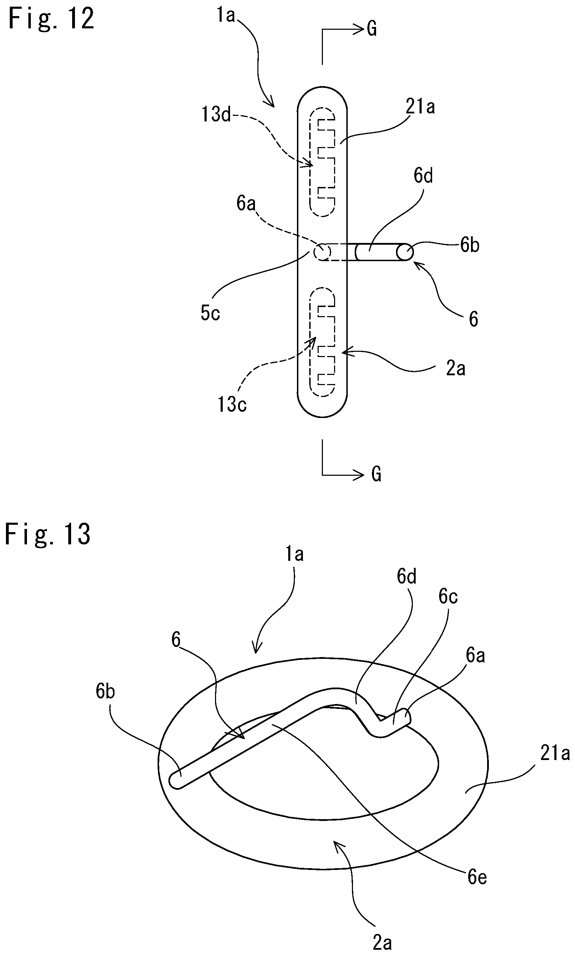

An organ prolapse preventing ring for intravaginal insertion according to the present invention may also be an organ prolapse preventing ring 1a for intravaginal insertion shown in FIGS. 10 to 18. The main difference between the organ prolapse preventing ring 1a of this embodiment and the organ prolapse preventing ring 1 for intravaginal insertion of the above embodiment is that the organ prolapse preventing ring 1a includes an elastic rod-shaped portion (string-shaped portion) 6 having one end 6a connected to a ring-shaped portion 2a. Arcuate reinforcing members 13a, 13b, 13c, and 13d used in the organ prolapse preventing ring 1a are almost the same as the arcuate reinforcing members 3a, 3b, 3c, and 3d described above, but the shape of recesses 72 is slightly different.

The organ prolapse preventing ring 1a of this embodiment includes the elastic rod-shaped portion 6 that has the one end 6a connected to a ring body 21a, another end 6b that is a free end, and an entire length longer than the inner diameter of the ring-shaped portion 2a. In this embodiment, the one end 6a of the elastic rod-shaped portion 6 is connected to the inner surface of the arcuate-reinforcing-member absent portion 5a. Also, the elastic rod-shaped portion 6 is formed integrally with the ring-shaped portion 2a with a soft material forming the ring body 21a as in this embodiment.

Furthermore, the one end 6a of the elastic rod-shaped portion 6 in the organ prolapse preventing ring 1a of this embodiment is located on the inner surface of the arcuate-reinforcing-member absent portion 5a, the elastic rod-shaped portion 6 includes a first portion 6c extending from the one end 6a toward the center of the ring-shaped portion 2a, a second portion 6d curved from an end of the first portion 6c and extending to a position above the surface of the ring-shaped portion 2a, and a third portion 6e curved from an end of the second portion 6d and extending above the ring-shaped portion 2a substantially in parallel with the ring-shaped portion 2a, and the end 6b of the third portion 6e is the free end.

Specifically, the elastic rod-shaped portion 6 is an elastic string-shaped member having a substantially circular cross section as shown in FIGS. 10 to 16. The diameter of the cross section of the elastic rod-shaped portion 6 is preferably 3 to 6 mm, and particularly preferably 3.5 to 5 mm. The one end 6a of the elastic rod-shaped portion 6 is connected to the inner surface of the arcuate-reinforcing-member absent portion 5a. The elastic rod-shaped portion 6 includes the first portion 6c extending from the one end 6a toward the center of the ring-shaped portion 2a with a predetermined length of about 5 to 15 mm. The elastic rod-shaped portion 6 also includes the second portion 6d that is curved from the end of the first portion 6c and extends obliquely to a position above the surface of the ring-shaped portion 2a in the direction toward the center. The end of the second portion 6d is closer to the arcuate-reinforcing-member absent portion 5c, which faces the arcuate-reinforcing-member absent portion 5a, than the center of the ring-shaped portion 2a is. Accordingly, the end side of the second portion 6d extends obliquely above the center of the ring-shaped portion 2a.

The elastic rod-shaped portion 6 also includes the third portion 6e extending from the end of the second portion 6d above the ring-shaped portion 2a substantially in parallel with the ring-shaped portion 2a. In this embodiment, the third portion 6e extends past a position above the arcuate-reinforcing-member absent portion 5c of the ring-shaped portion 2a. Accordingly, the third portion 6e extends past the outer edge of the ring-shaped portion 2a by a predetermined length of about 5 to 20 mm. The end 6b of the third portion 6e is the free end and has a semi-spherical shape including no corner.

When such an elastic rod-shaped portion 6 is provided, it is easy to confirm that the organ prolapse preventing ring 1a has been inserted into the vagina, and the organ prolapse preventing ring 1a can be easily taken out from the vagina.

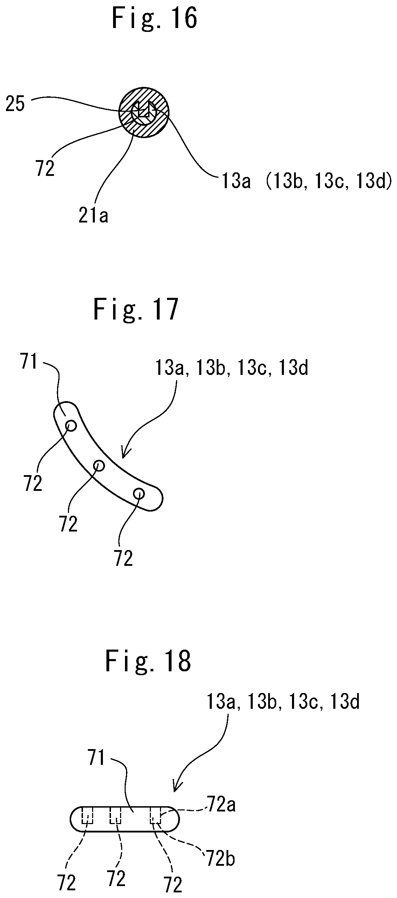

As shown in FIGS. 17 and 18, the basic configuration of the arcuate reinforcing members 13a, 13b, 13c, and 13d used in the organ prolapse preventing ring 1a is the same as the configuration of the arcuate reinforcing members 3a, 3b, 3c, and 3d described above, but the shape of the recesses 72 provided in a body 71 is slightly different. The entirety of each recess 72 in the arcuate reinforcing members 13a, 13b, 13c, and 13d is formed by a main portion 72a extending with substantially the same outer diameter, and a bottom 72b of the recess 72 is a flat surface.

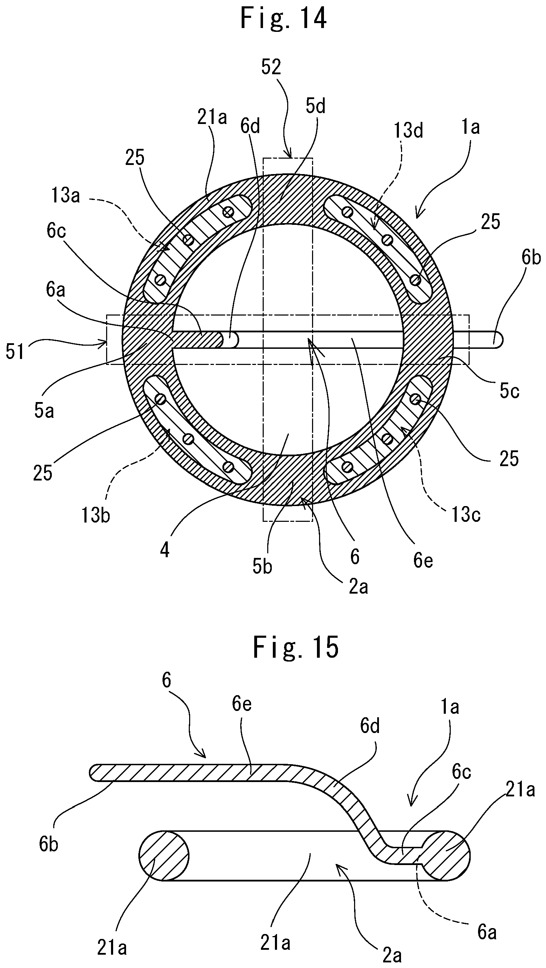

As shown in FIGS. 14 and 16, the recesses 72 are filled with the soft material 25 forming the ring body. The diameter of each recess 72 is preferably 2/10 to 6/10 of the diameter of the arcuate reinforcing members 13a, 13b, 13c, and 13d, and particularly preferably 3/10 to 5/10 of the diameter of the arcuate reinforcing members 13a, 13b, 13c, and 13d. The entirety of each recess 72 extends with the same diameter. The depth of each recess 72 is preferably 40/100 to 75/100 of the diameter of the arcuate reinforcing members 13a, 13b, 13c, and 13d, and particularly preferably 45/100 to 70/100 of the diameter of the arcuate reinforcing members 13a, 13b, 13c, and 13d.

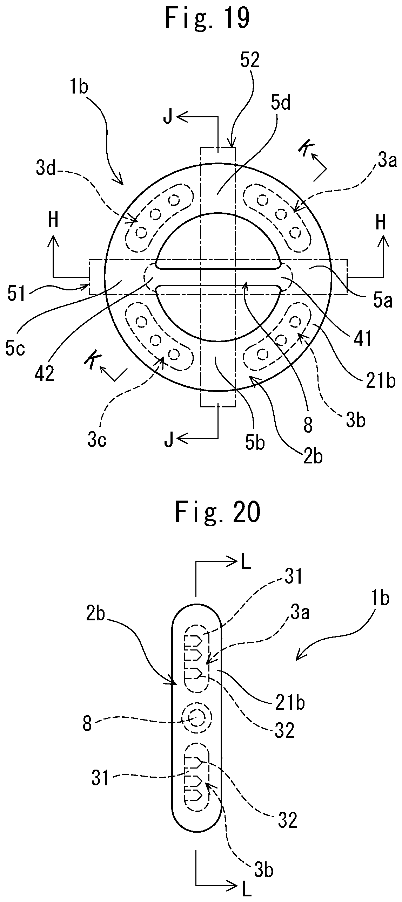

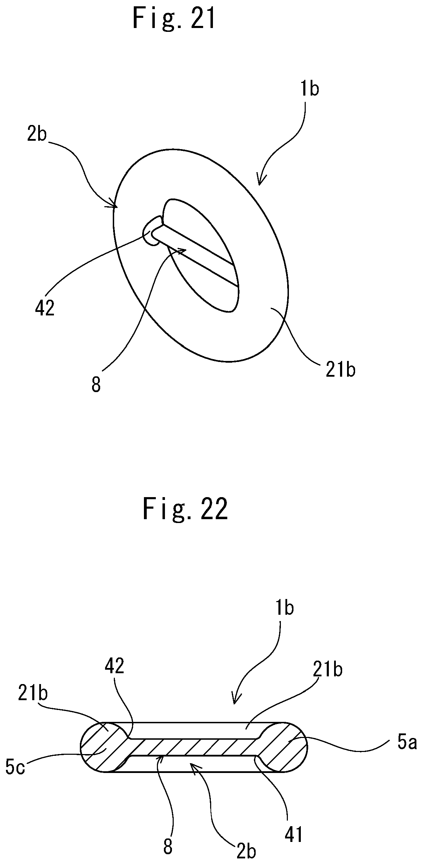

An organ prolapse preventing ring for intravaginal insertion according to the present invention may also be an organ prolapse preventing ring 1b for intravaginal insertion shown in FIGS. 19 to 25. The main difference between the organ prolapse preventing ring 1b of this embodiment and the organ prolapse preventing ring 1 for intravaginal insertion of the above embodiment is that the organ prolapse preventing ring 1b includes an elastic rod-shaped portion (string-shaped portion) 8 having one end 41 and another end 42 that are connected to a ring-shaped portion 2b.

The organ prolapse preventing ring 1b of this embodiment includes the elastic rod-shaped portion (string-shaped portion) 8 having the one end 41 and the other end 42 and extending so as to pass through the center of the ring-shaped portion 2b. Specifically, the one end 41 of the elastic rod-shaped portion 8 is connected to the inner surface of the arcuate-reinforcing-member absent portion 5a. The elastic rod-shaped portion 8 extends linearly and passes through the center of the ring-shaped portion 2b, and the other end thereof is connected to the inner surface of the arcuate-reinforcing-member absent portion 5c facing the arcuate-reinforcing-member absent portion 5a. The elastic rod-shaped portion 8 is formed integrally with the ring-shaped portion 2b with a soft material forming a ring body 21b.

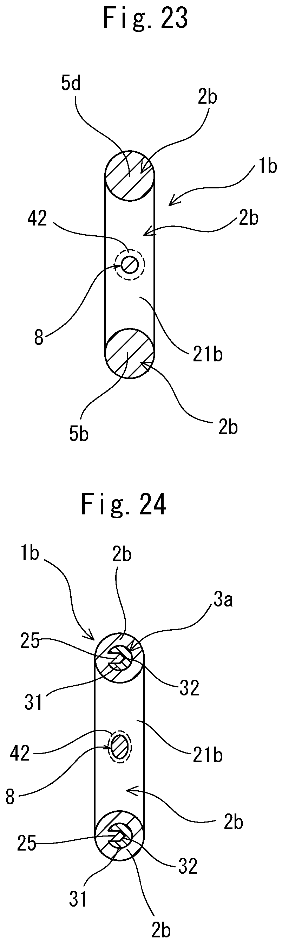

As shown in FIGS. 23 and 24, the elastic rod-shaped portion 8 is an elastic string-shaped member having a substantially circular cross section. The diameter of the cross section of the elastic rod-shaped portion 8 is preferably 3 to 6 mm, and particularly preferably 3.5 to 5 mm.

An organ prolapse preventing ring for intravaginal insertion according to the present invention includes a ring-shaped portion formed by a frame portion having a circular or oval cross section. The ring-shaped portion includes a ring body formed of a soft material and four arcuate reinforcing members formed of a hard or semi-hard material and embedded in the ring body. The four arcuate reinforcing members are spaced apart from each other. The ring-shaped portion includes a first pair of arcuate-reinforcing-member absent portions that are formed of only the soft material and face each other, a first bendable portion composed of the first pair of arcuate-reinforcing-member absent portions, a second pair of arcuate-reinforcing-member absent portions that are formed of only the soft material, orthogonal to the first pair of arcuate-reinforcing-member absent portions, and face each other, and a second bendable portion composed of the second pair of arcuate-reinforcing-member absent portions.

In particular, the organ prolapse preventing ring for intravaginal insertion according to the present invention includes the first bendable portion and the second bendable portion that can be bent in a direction different from the bendable direction of the first bendable portion, or more specifically, in a direction substantially orthogonal to the bendable direction of the first bendable portion. Accordingly, the organ prolapse preventing ring can be bent in both of the two bendable portions, and therefore, the bending position can be easily selected and the bending operation can be easily performed. Moreover, two side portions of the ring project in the bent state, and the ring can be inserted into the vagina from one of the two side portions, and therefore, the ring can be easily inserted into the vagina. Furthermore, the ring-shaped portion is formed by the frame portion having a circular or oval cross section, and therefore, it is possible to favorably take out the ring from the vagina by hooking a finger on the ring-shaped portion.

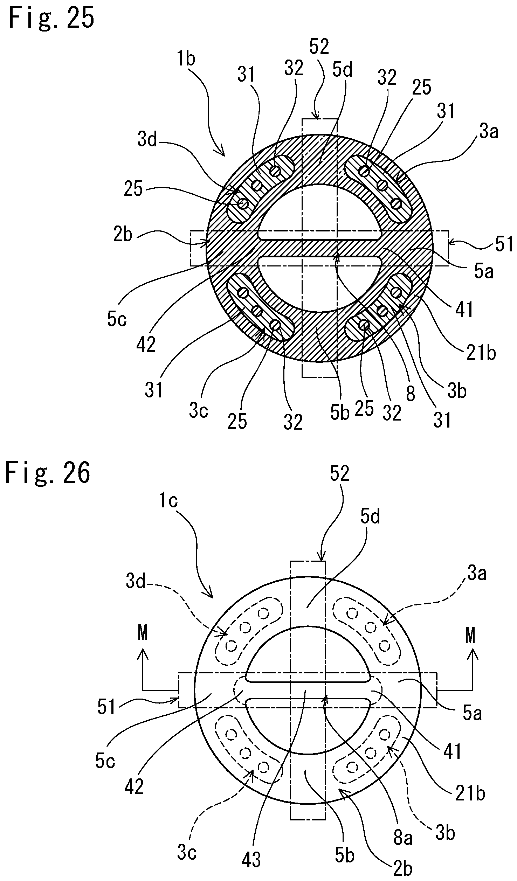

An organ prolapse preventing ring for intravaginal insertion according to the present invention may be an organ prolapse preventing ring 1c for intravaginal insertion shown in FIGS. 26 to 30. The main difference between the organ prolapse preventing ring 1c of this embodiment and the organ prolapse preventing ring 1 for intravaginal insertion of the above embodiment is that the organ prolapse preventing ring 1c includes an elastic rod-shaped portion (string-shaped portion) 8a having one end 41 and another end 42 that are connected to the ring-shaped portion 2b.

The organ prolapse preventing ring 1c of this embodiment includes the elastic rod-shaped portion (string-shaped portion) 8a having the one end 41 and the other end 42 that are connected to the ring-shaped portion 2b. Specifically, the one end 41 of the elastic rod-shaped portion 8a is connected to the inner surface of the arcuate-reinforcing-member absent portion 5a. Similarly, the other end 42 of the elastic rod-shaped portion 8a is connected to the inner surface of the arcuate-reinforcing-member absent portion 5c facing the arcuate-reinforcing-member absent portion 5a. The elastic rod-shaped portion 8a includes a curved portion 43 as its central portion.

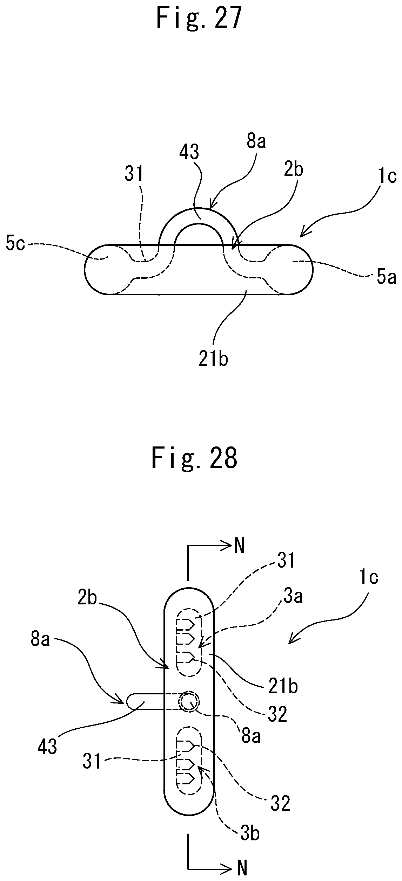

It is preferable that the curved portion 43 has a top above the center of the ring-shaped portion 2b, and it is particularly preferable that the curved portion 43 is curved in a substantially semi-circular shape. It is preferable that the top of the curved portion 43 is located above a central part of the ring-shaped portion 2b. Note that the curved portion 43 may be bent at the top, or a top portion of the curved portion 43 may extend linearly a predetermined length, or the entire curved portion 43 may be trapezoidal. As shown in FIGS. 27, 28, and 29, in the organ prolapse preventing ring 1c of this embodiment, a central part of the curved portion 43 projects upward from a circular surface formed by the upper surface (the surface on the side where the curved portion extends) of the ring-shaped portion 2b.

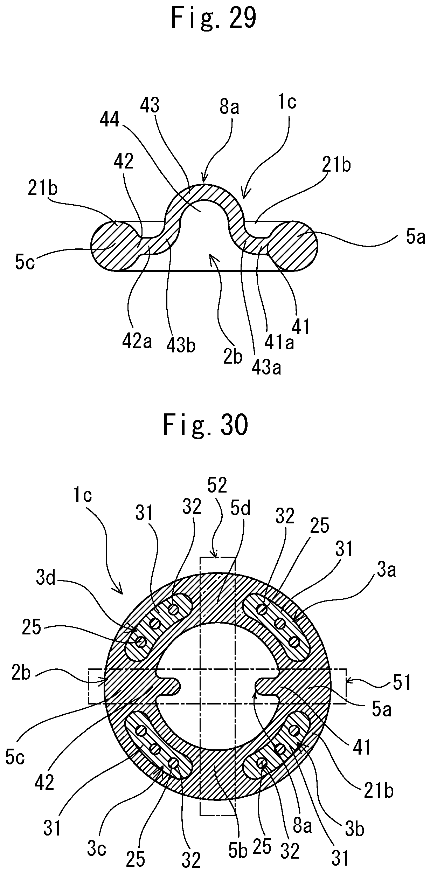

As shown in FIG. 29, in the organ prolapse preventing ring 1c of this embodiment, the elastic rod-shaped portion 8a includes a short linear portion 41a extending inward (toward the center) of the ring-shaped portion 2b from the one end 41, and the one-end side curved portion 43a curved from the short one-end side linear portion 41a and extending in a direction orthogonal to the linear portion 41a. The elastic rod-shaped portion 8a also includes a short other-end side linear portion 42a extending inward (toward the center) of the ring-shaped portion 2b from the other end 42, and the other-end side curved portion 43b curved from the short other-end side linear portion 42a and extending in a direction orthogonal to the linear portion 42a (in the same direction as the direction in which the one-end side curved portion 43a extends). The curved portion 43 connects the one-end side curved portion 43a and the other-end side curved portion 43b.

The central part of the curved portion 43 projects upward from the circular surface formed by the upper surface (the surface on the side where the curved portion extends) of the ring-shaped portion 2b. Also, in this embodiment, the curved portion 43 forms a space 44 on the inner side thereof (the side where there is the center of the ring-shaped portion 2b) above the center of the ring-shaped portion 2b.

The elastic rod-shaped portion 8a is formed integrally with the ring-shaped portion 2b with a soft material forming the ring body 21b. The elastic rod-shaped portion 8a is an elastic string-shaped member having a substantially circular cross section as shown in FIG. 28. The diameter of the cross section of the elastic rod-shaped portion 8a is preferably 3 to 6 mm, and particularly preferably 3.5 to 5 mm.

The organ prolapse preventing ring 1c for intravaginal insertion of this embodiment includes the elastic rod-shaped portion 8a including the curved portion 43 as its central portion, so that it is possible to favorably take out the ring 1c from the vagina by hooking a finger on the curved portion.

An organ prolapse preventing ring for intravaginal insertion according to the present invention includes a ring-shaped portion formed by a frame portion having a circular or oval cross section. The ring-shaped portion includes a ring body formed of a soft material and four arcuate reinforcing members formed of a hard or semi-hard material and embedded in the ring body. The four arcuate reinforcing members are spaced apart from each other. The ring-shaped portion includes a first pair of arcuate-reinforcing-member absent portions that are formed of only the soft material and face each other, a first bendable portion composed of the first pair of arcuate-reinforcing-member absent portions, a second pair of arcuate-reinforcing-member absent portions that are formed of only the soft material, orthogonal to the first pair of arcuate-reinforcing-member absent portions, and face each other, and a second bendable portion composed of the second pair of arcuate-reinforcing-member absent portions.

In particular, the organ prolapse preventing ring includes the first bendable portion and the second bendable portion that can be bent in a direction different from the bendable direction of the first bendable portion. Accordingly, the organ prolapse preventing ring can be bent in both of the two bendable portions, and therefore the bending operation can be easily performed. Moreover, two side portions of the ring project in the bent state, and the ring can be inserted into the vagina from one of the two side portions, and therefore, the ring can be easily inserted into the vagina. Furthermore, the ring-shaped portion is formed by the frame portion having a circular or oval cross section, and therefore, it is possible to favorably take out the ring from the vagina by hooking a finger on the ring-shaped portion.

### Industrial Applicability

An organ prolapse preventing ring according to the present invention is as follows.
(1) An organ prolapse preventing ring for intravaginal insertion, the ring comprising:
   a ring-shaped portion formed by a frame portion having a circular or oval cross section,
   wherein the ring-shaped portion includes a ring body formed of a soft material and four arcuate reinforcing members formed of a hard or semi-hard material and embedded in the ring body, the four arcuate reinforcing members are spaced apart from each other, and the ring-shaped portion includes a first pair of arcuate-reinforcing-member absent portions that are formed of only the soft material and face each other, a first bendable portion composed of the first pair of arcuate-reinforcing-member absent portions, a second pair of arcuate-reinforcing-member absent portions that are formed of only the soft material, orthogonal to the first pair of arcuate-reinforcing-member absent portions, and face each other, and a second bendable portion composed of the second pair of arcuate-reinforcing-member absent portions.

This organ prolapse preventing ring for intravaginal insertion includes the first bendable portion and the second bendable portion that can be bent in a direction different from the bendable direction of the first bendable portion. Accordingly, the organ prolapse preventing ring can be bent in both of the two bendable portions, and therefore, the bending operation can be easily performed. Moreover, two side portions of the ring project in the bent state, and the ring can be inserted into the vagina from one of the two side portions, and therefore, the ring can be easily inserted into the vagina. Furthermore, the ring-shaped portion is formed by the frame portion having a circular or oval cross section, and therefore, it is possible to favorably take out the ring from the vagina by hooking a finger on the ring-shaped portion.

Also, the above embodiment may be as follows.
(2) The organ prolapse preventing ring according to the above (1),
   wherein each of the arcuate reinforcing members includes a recess extending from one surface side toward another surface side of the ring-shaped portion, and the recess is filled with the soft material forming the ring body.
(3) The organ prolapse preventing ring according to the above (1) or (2), wherein the arcuate reinforcing members are arranged at approximately equal angular intervals with respect to a center of the ring-shaped portion, a central angle of an arc formed by each of the arcuate reinforcing members is 40 to 72 degrees, and a central angle of an arc formed by each of the four arcuate-reinforcing-member absent portions is 24 to 45 degrees.
(4) . The organ prolapse preventing ring according to the above (1) or (2),
   wherein the arcuate reinforcing members are arranged at approximately equal angular intervals with respect to a center of the ring-shaped portion, a central angle of an arc formed by each of the arcuate reinforcing members is 45 to 65 degrees, and a central angle of an arc formed by each of the four arcuate-reinforcing-member absent portions is 25 to 40 degrees.
(5) The organ prolapse preventing ring according to the above (2), wherein each of the arcuate reinforcing members includes the recess in each of two end portions.
(6) The organ prolapse preventing ring according to any one of the above (1) to (5), wherein the soft material forming the four arcuate-reinforcing-member absent portions is an elastic material having a durometer hardness A of 15 to 50.
(7) The organ prolapse preventing ring according to any one of the above (1) to (6), wherein the soft material forming the ring body is soft rubber or a soft elastomer.
(8) The organ prolapse preventing ring according to any one of the above (1) to (7), further comprising: an elastic rod-shaped portion having one end connected to the ring body, another end that is a free end, and an entire length longer than an inner diameter of the ring-shaped portion.
(9) The organ prolapse preventing ring according to the above (8), wherein the one end of the elastic rod-shaped portion is connected to one of the arcuate-reinforcing-member absent portions.
(10) The organ prolapse preventing ring according to the above (8) or (9), wherein the elastic rod-shaped portion is formed integrally with the ring-shaped portion with the soft material forming the ring body.
(11) The organ prolapse preventing ring according to any one of the above (8) to (10), wherein the one end of the elastic rod-shaped portion is located on an inner surface of one of the arcuate-reinforcing-member absent portions, the elastic rod-shaped portion includes a first portion extending from the one end toward a center of the ring-shaped portion, a second portion curved from an end of the first portion and extending to a position above a surface of the ring-shaped portion, and a third portion curved from an end of the second portion and extending above the ring-shaped portion substantially in parallel with the ring-shaped portion, and an end of the third portion is the free end.
(12) The organ prolapse preventing ring according to any one of the above (1) to (7),claims 1 to 7, further comprising: an elastic rod-shaped portion having one end and another end that are connected to the ring body.
(13) The organ prolapse preventing ring according to the above (12), wherein the one end and the other end of the elastic rod-shaped portion are connected to an inner surface of the ring body.
(14) The organ prolapse preventing ring according to the above (12) or (13), wherein the one end of the elastic rod-shaped portion is connected to an inner surface of one of the arcuate-reinforcing-member absent portions, and the other end of the elastic rod-shaped portion is connected to an inner surface of another one of the arcuate-reinforcing-member absent portions facing the arcuate-reinforcing-member absent portion to which the one end is connected.
(15) The organ prolapse preventing ring according to any one of the above (12) to (14), wherein the elastic rod-shaped portion linearly extends from the one end to the other end.
(16) The organ prolapse preventing ring according to any one of the above (12) to (14) wherein the elastic rod-shaped portion includes a curved portion having a top above a center of the ring-shaped portion.

## Claims

1. An organ prolapse preventing ring for intravaginal insertion, the ring comprising:
a ring-shaped portion formed by a frame portion having a circular or oval cross section,
wherein the ring-shaped portion includes a ring body formed of a soft material and four arcuate reinforcing members formed of a hard or semi-hard material and embedded in the ring body, the four arcuate reinforcing members are spaced apart from each other, and the ring-shaped portion includes a first pair of arcuate-reinforcing-member absent portions that are formed of only the soft material and face each other, a first bendable portion composed of the first pair of arcuate-reinforcing-member absent portions, a second pair of arcuate-reinforcing-member absent portions that are formed of only the soft material, orthogonal to the first pair of arcuate-reinforcing-member absent portions, and face each other, and a second bendable portion composed of the second pair of arcuate-reinforcing-member absent portions.

2. The organ prolapse preventing ring according to claim 1,
wherein each of the arcuate reinforcing members includes a recess extending from one surface side toward another surface side of the ring-shaped portion, and the recess is filled with the soft material forming the ring body.

3. The organ prolapse preventing ring according to claim 1 or 2,
wherein the arcuate reinforcing members are arranged at approximately equal angular intervals with respect to a center of the ring-shaped portion, a central angle of an arc formed by each of the arcuate reinforcing members is 40 to 72 degrees, and a central angle of an arc formed by each of the four arcuate-reinforcing-member absent portions is 24 to 45 degrees.

4. The organ prolapse preventing ring according to claim 1 or 2,
wherein the arcuate reinforcing members are arranged at approximately equal angular intervals with respect to a center of the ring-shaped portion, a central angle of an arc formed by each of the arcuate reinforcing members is 45 to 65 degrees, and a central angle of an arc formed by each of the four arcuate-reinforcing-member absent portions is 25 to 40 degrees.

5. The organ prolapse preventing ring according to claim 2,
wherein each of the arcuate reinforcing members includes the recess in each of two end portions.

6. The organ prolapse preventing ring according to any one of the above claim 1 to 5,
wherein the soft material forming the four arcuate-reinforcing-member absent portions is an elastic material having a durometer hardness A of 15 to 50.

7. The organ prolapse preventing ring according to any one of the above claim 1 to 6,
wherein the soft material forming the ring body is soft rubber or a soft elastomer.

8. The organ prolapse preventing ring according to any one of the above claim 1 to 7, further comprising:
an elastic rod-shaped portion having one end connected to the ring body, another end that is a free end, and an entire length longer than an inner diameter of the ring-shaped portion.

9. The organ prolapse preventing ring according to claim 8,
wherein the one end of the elastic rod-shaped portion is connected to one of the arcuate-reinforcing-member absent portions.

10. The organ prolapse preventing ring according to claim 8 or 9,
wherein the elastic rod-shaped portion is formed integrally with the ring-shaped portion with the soft material forming the ring body.

11. The organ prolapse preventing ring according to any one of the above claim 8 to 10,
wherein the one end of the elastic rod-shaped portion is located on an inner surface of one of the arcuate-reinforcing-member absent portions, the elastic rod-shaped portion includes a first portion extending from the one end toward a center of the ring-shaped portion, a second portion curved from an end of the first portion and extending to a position above a surface of the ring-shaped portion, and a third portion curved from an end of the second portion and extending above the ring-shaped portion substantially in parallel with the ring-shaped portion, and an end of the third portion is the free end.

12. The organ prolapse preventing ring according to any one of the above claim 1 to 7, further comprising:
an elastic rod-shaped portion having one end and another end that are connected to the ring body.

13. The organ prolapse preventing ring according to claim 12,
wherein the one end and the other end of the elastic rod-shaped portion are connected to an inner surface of the ring body.

14. The organ prolapse preventing ring according to claim 12 or 13,
wherein the one end of the elastic rod-shaped portion is connected to an inner surface of one of the arcuate-reinforcing-member absent portions, and the other end of the elastic rod-shaped portion is connected to an inner surface of another one of the arcuate-reinforcing-member absent portions facing the arcuate-reinforcing-member absent portion to which the one end is connected.

15. The organ prolapse preventing ring according to any one of the above claim 12 to 14,
wherein the elastic rod-shaped portion linearly extends from the one end to the other end.

16. The organ prolapse preventing ring according to any one of the above claim 12 to 14,
wherein the elastic rod-shaped portion includes a curved portion having a top above a center of the ring-shaped portion.
